# EUROPEAN PATENT APPLICATION

(11) **EP 3 928 662 A1**
(43) Date of publication of application: **29.12.2021**
(21) Application number: 20759921.8
(22) Date of filing: 21.02.2020
(51) Int. Cl.: A47C 7/72, A47C 9/00, H04R 1/00, H04R 1/02, H04R 3/00, A61H 23/02, G10K 15/00

(54) **HUMAN BODY SUPPORT DEVICE**

(30) Priority: 22.02.2019 JP 2019030196
(71) Applicant: Japan Tobacco Inc., Tokyo 105-6927 (JP); Enhance Experience Inc., Mountain View CA 94043 (US); Flowplateaux Co., Ltd., Meguro-ku Tokyo 153-0043 (JP)
(72) Inventor: SAKUMA, Sayako, Tokyo 130-8603 (JP); MIZUGUCHI, Tetsuya, Mountain View, California 94043 (US); SATO, Ayahiko, Tokyo 153-0043 (JP)
(74) Representative: Isarpatent
(86) International application number: PCT/JP2020/006983
(87) International publication number: WO 2020/171195

(57) **Abstract**

The present invention makes it possible to provide a user with a new sensation through a tactile sensation, an auditory sensation, and a visual sensation. A human body support device 1 has an overall shape that can easily be used as a chair or a couch. The human body support device 1 comprises a frame that is formed from, for example, metal or resin, and a plurality of modules that are arranged in a plurality of sites to support the user body on the frame. These modules include a sound emitting module M1 that is provided with a speaker 70, and a vibrating and light-emitting module M2 that is provided with an LED 30 and a voice coil motor 50. These modules have an elastic part that is formed by an elastic material such as a sponge in a location that is in contact with the user body. The orientation and position of the modules are designed so as to be in proper contact with and support the user body, and accordingly, the user body can be comfortably supported.

## Description

### TECHNICAL FIELD

The present invention relates to a technique for stimulating the five senses of a user.

### BACKGROUND ART

For example, Patent Literature 1 discloses a construction for making plural vibration units, which are installed in an easy-chair, vibrate based on a reproduced signal of musical sound.

### CITATION LIST

### PATENT LITERATURE

PTL 1: Japanese Patent Application Public Disclosure No. H07-222781

### SUMMARY OF INVENTION

### TECHNICAL PROBLEM

An object of the present invention is to provide a user with a new sensation, based on a tactile sensation, an auditory sensation, and a visual sensation.

### SOLUTION TO PROBLEM

For solving the above problem, the present invention provides a human body support device which is characterized in that it comprises: a frame, and plural modules which are arranged in plural parts, in the frame, which support a body of a user; wherein each of the plural modules comprises an elastic part which is arranged in a position where the body of the user is brought in contact with the module; and, in the plural modules, each of at least one or more sound emitting modules is provided with a sound emitting device, and each of at least two or more vibrating and light-emitting modules is provided with a vibrating device and a light emitting device.

It may be possible to provide an information processing device which controls the sound emitting device, the vibrating device, and the light emitting device in a synchronizing manner.

The information processing device may control the vibrating device or the light emitting device to vibrate or emit light in different manners.

The information processing device may perform switching of a mode between a first operation mode for performing light emission accompanied with vibration in a state that the body of a user is being supported, and a second operation mode for performing light emission without vibration in a state that the body of a user is not being supported.

Each of the vibrating and light-emitting modules may comprise a vibration suppressing mechanism for suppressing propagation of vibration from the vibrating device installed in the vibrating and light-emitting module to the frame.

The respective modules may be arranged in such a manner that, in the frame, the respective modules do not come in contact with each other.

The frame may comprise a first frame which extends in the direction that the backbone of a user extends when the body of the user is supported, and plural second frames which intersect the first frame, and the respective modules may be arranged in the second frames.

The at least one or more vibrating and light-emitting modules may be attachable/detachable to/from the frame; and, in the vibrating and light-emitting module, when the vibrating and light-emitting module is in the state that it is being connected to the frame, the vibrating device may perform vibration or the light emitting device may perform light emission by using electric power supplied via the frame, and, when the vibrating and light-emitting module is in the state that it is being detached from the frame, the vibrating device may perform vibration or the light emitting device may perform light emission by using electric power supplied from a battery included in the vibrating and light-emitting module.

The shapes of the respective modules may be approximately the same with each other.

The sizes of the respective vibrating and light-emitting modules are approximately the same with each other.

### ADVANTAGEOUS EFFECTS OF INVENTION

According to the present invention, it becomes possible to provide a user with a new sensation, based on a tactile sensation, an auditory sensation, and a visual sensation.

### BRIEF DESCRIPTION OF DRAWINGS

Fig. 1 is a figure showing an example of an electrical structure of a human body support device according to an embodiment of the present invention.
Fig. 2 is a block diagram showing an example of an electrical structure of an information processing device.
Fig. 3 (A) is a block diagram showing an example of an electrical structure of a sound emitting module, and Fig. 3 (B) is a block diagram showing an example of an electrical structure of a vibrating and light-emitting module.
Fig. 4 is a side view of the human body support device.
Fig. 5 is a top view of the human body support device.
Fig. 6 is a front view of the human body support device.
Fig. 7 is a rear-side view of the human body support device.
Fig. 8 is a perspective view showing a state that a user is sitting in the human body support device.
Fig. 9 is an exploded perspective view of the vibrating and light-emitting module.

### DESCRIPTION OF EMBODIMENTS

### [Embodiments]

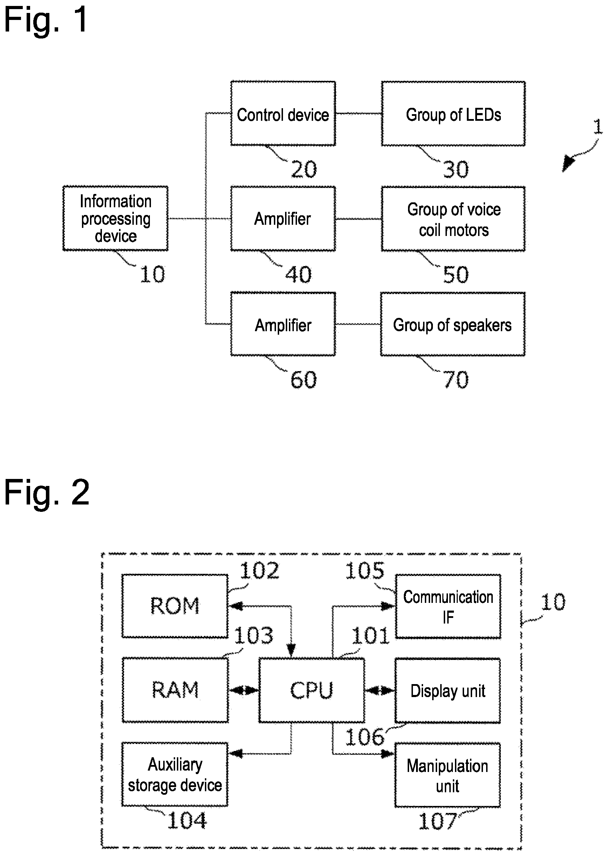

First, an electrical structure of a human body support device 1 according to an embodiment of the present invention will be explained. As shown in Fig. 1, the human body support device 1 comprises an information processing device 10 such as a personal computer or the like, a control device 20 connected to the information processing device 10, a group of LEDs (Light Emitting Diodes) 30 connected to the control device 20, an amplifier 40 connected to the information processing device 10, a group of voice coil motors 50 connected to the amplifier 40, an amplifier 60 connected to the information processing device 10, and a group of speakers 70 connected to the amplifier 60. The LED 30 is an example of a light emitting device, the voice coil motor 50 is an example of a vibrating device, and the speaker 70 is and example of a sound emitting device; however, the light emitting device, the vibrating device, and the sound emitting device relating to the present invention are not limited to those illustrated in Fig. 1. The respective devices illustrated in Fig. 1 may be communicably connected each other in a wired manner, or may be communicably connected each other in a wireless manner.

Fig. 2 is a figure showing an example of an electrical structure of the information processing device 10. The information processing device 10 comprises a CPU (Central Processing Unit) 101, a ROM (Read Only Memory) 102, a RAM (Random Access Memory) 103, an auxiliary storage device 104, a communication IF (interface) 105, a display unit 106, and a manipulation unit 107.

The CPU 101 is a processor which performs various kinds of operation. The ROM 102 is a nonvolatile memory which stores, for example, a program and data that are used when activating the information processing device 10. The RAM 103 is a volatile memory which functions as a work area that is used by the CPU 101 when it executes a program. The auxiliary storage device 104 is a nonvolatile storage device such as a HDD (Hard Disk Drive), a SSD (Solid State Drive), or the like, for example, and stores programs and data used in the information processing device 10. The communication IF 105 is an interface which is used for communication that is performed in accordance with a predetermined communication standard. The communication standard may be a standard for wired communication, or may be a standard for wireless communication. In the case of wireless communication, the communication IF 105 comprises an antenna and a communication circuit which operate in accordance with a communication standard such as Wi-Fi (a registered trademark) or the like, for example. The display unit 106 comprises, for example, a liquid crystal panel and a liquid crystal driving circuit, and displays an image corresponding to image data. The manipulation unit 107 comprises manipulation elements such as a key, a touch sensor, and so on, for example, accepts manipulation performed by a user, and supplies a signal corresponding to the manipulation to the CPU 101. It should be reminded that, in addition to the constructions illustrated in Fig. 2, the information processing device 10 may comprise other constructions such as a voice input/output unit and so on, for example.

More specifically, the auxiliary storage device 104 stores musical-sound control data for making musical sound be emitted from the group of speakers 70, light-emission control data for making light be emitted from the group of LEDs 30 in synchronous with emission of the musical sound, vibration control data for making the group of voice coil motors 50 vibrate in synchronous with emission of the musical sound, and a software program comprising an algorithm for performing control using the above data. The CPU 101 in the information processing device 10 executes the above software program to thereby control, via the control device 20, timing of emission of light, time of emission of light, and so on from the group of LEDs 30, control, via the amplifier 40, the frequency and amplitude of vibration and so on of the group of voice coil motors 50, and control, via the amplifier 60, emission of musical sound from the group of speakers 70. That is, the CPU 101 in the information processing device 10 controls the group of LEDs 30 functioning as a light emitting device, the group of voice coil motors 50 functioning as a vibrating device, and the group of speakers 70 functioning as a musical sound emitting device in such a manner that they are operated synchronously with each other. For example, it is controlled in such a manner that, when the rhythm of musical sound emitted from the group of speakers 70 is rapid, the group of voice coil motors 50 is made to vibrate vigorously and intervals between light emissions by the group of LEDs 30 are made shorter, and, on the other hand, when the rhythm of musical sound emitted from the group of speakers 70 is slow, the group of voice coil motors 50 is made to vibrate softly and intervals between light emissions by the group of LEDs 30 are made longer.

In this regard, the CPU 101 in the information processing device 10 may control respective LEDs 30 in the LED group and the respective voice coil motors 50 in the voice coil motor group in different manners. For example, the CPU 101 in the information processing device 10 may perform controlling of light emission with respect to one LED 30 and controlling of light emission with respect to the other LED 30, separately. Further, the CPU 101 in the information processing device 10 may perform controlling of vibration with respect to one voice coil motor 50 and controlling of vibration with respect to the other voice coil motor 50, separately.

The algorithm of software stored in the auxiliary storage device 104 is an algorithm for giving a stimulus to a user's auditory sensation by musical sound emitted from the group of speakers 70, and also giving stimuluses to the user's tactile and visual sensations by vibration applied by the group of voice coil motors 50 and light emission from the group of LEDs 30. By receiving stimuluses through the auditory sensation, the tactile sensation, and the visual sensation, a user can feel a new sensation that has not yet experienced, wherein, in the new sensation, states such as a comfortable feeling state, a pleasant feeling state, a relaxation feeling state, an excitement feeling state, and so on, for example, are continuously changed from one to the other, or the above states are mixed with one another. For example, a sensation that forces a person to visualize a color and/or light and see it when the person listens to a sound, that is, a phenomenon wherein a single stimulation induces, in addition to a sensation that is originally induced thereby, a sensation in a different field, is referred to as "synesthesia," and a user can have a pseudo experience of synesthesia, by the effect provided by the human body support device 1.

Further, the operation modes of the information processing device 10 comprise two kinds of operation modes. One is a first operation mode wherein, in the state that the body of a user is being supported by the human body support device 1, light is emitted and vibration is added during when musical sound is emitted; and the other is a second operation mode wherein, in the state that the body of a user is not supported by the human body support device 1, light is emitted without addition of vibration. The first operation mode is an operation mode for providing a user with a special sensation by applying stimulations to the user, mainly through the auditory sensation stimulated by emitted musical sound, the tactile sensation stimulated by vibration, and the visual sensation stimulated by emitted light. On the other hand, the second operation mode is an operation mode wherein the human body support device 1 functions as an interior article or an object which emits light, and a user can enjoy the light emitting state thereof through the user's visual sensation. In the second operation mode, emission of musical sound may or may not be performed.

The CPU 101 in the information processing device 10 changes the mode between the above operation modes in accordance with manipulation performed by a user, for example. Alternatively, for example, the human body support device 1 may be provided with a sensor for detecting the state that the body of a user is being in contact therewith, and may be constructed in such a manner that the CPU 101 operates in the first operation mode when it is judged by the sensor that the state is that the body of the user is being supported by the human body support device 1, and the CPU 101 operates in the second operation mode when it is judged by the sensor that the state is that the body of the user is not being supported by the human body support device 1.

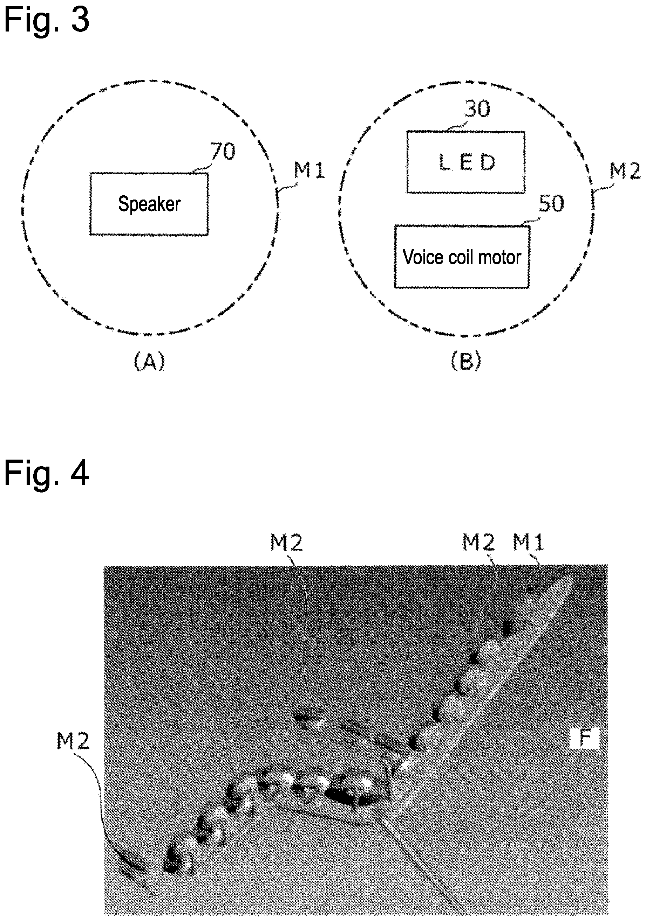

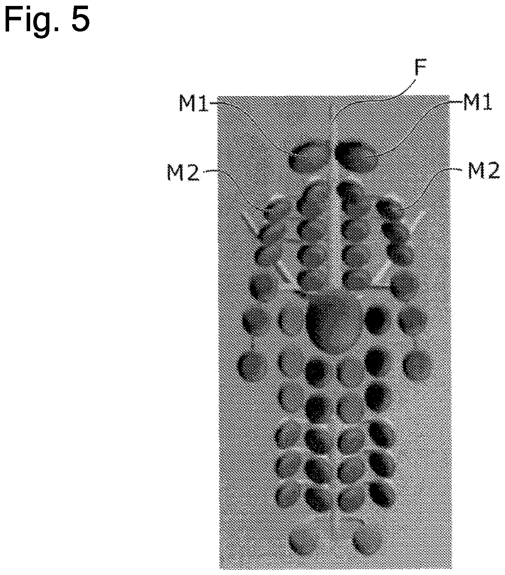

Next, the external appearance and the construction of the human body support device 1 will be explained with reference to Figs. 3-9. The human body support device 1 comprises, for example, a frame constructed by using metal and/or resin, and plural modules which are arranged in plural parts, which support the body of a user, of the frame. The above modules comprise two kinds of modules which are referred to as a sound emitting module M1 which is provided with a speaker 70 as shown in Fig. 3 (A), and a vibrating and light-emitting module M2 which is provided with an LED 30 and a voice coil motor 50 as shown in Fig. 3 (B).

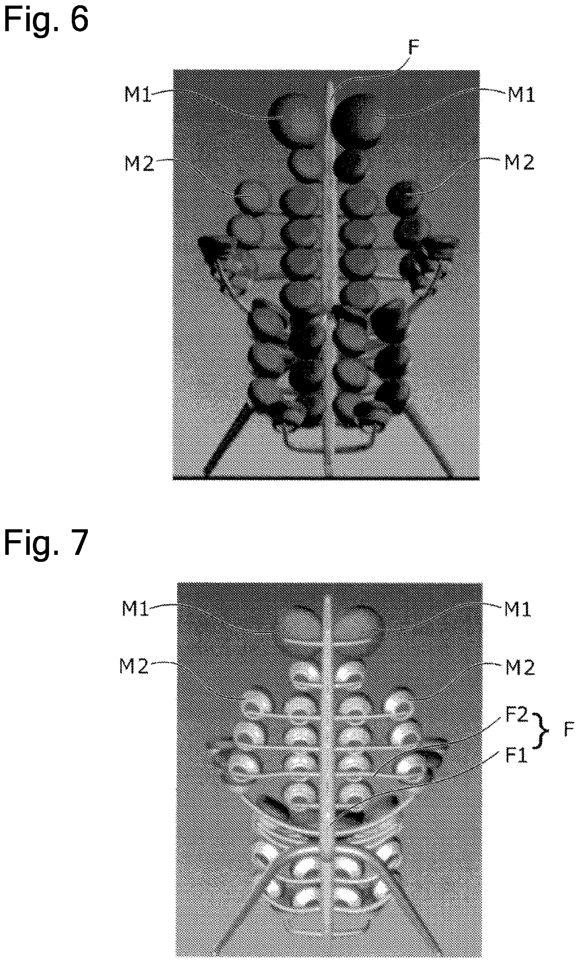

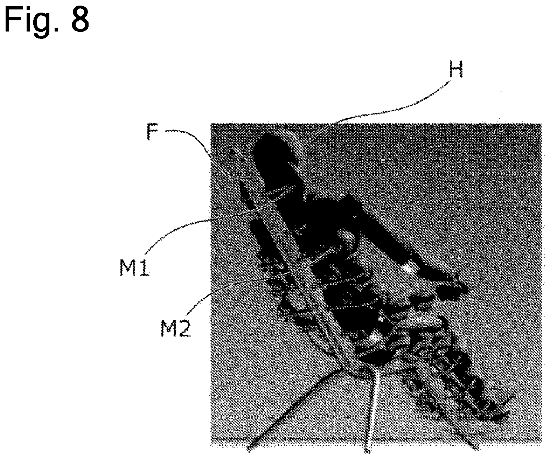

The human body support device 1 is constructed to have a shape that makes the device be used easily as a chair or a couch by a user H (refer to Fig. 8). A frame F comprises a first frame F1 which extends in the direction that the backbone of a user extends when the user sits in the human body support device 1, and plural second frames F2 which intersect an axis in the direction that the frame F1 extends (typically, orthogonally intersect at intersections thereof) (refer to Fig. 7). The above frames F1 and F2 are constructed to have curves that fit the body of a user. In the inside of the respective frames F1 and F2, wires for electrically connecting the information processing device 10 with the group of LEDs 30, the group of voice coil motors 50, and the group of speakers 70 are installed. Electric power is supplied to the group of LEDs 30, the group of voice coil motors 50, and the group of speakers 70 via wires installed in the frame F.

Respective modules M1 and M2, especially, those in the second frame F2, are arranged to be bilaterally symmetrical when they are viewed from the front. With respect to a second frame F2 which is installed in the highest position (a position that is close to the head of a user when the user is supported by the human body support device 1), a pair of sound emitting modules M1 are installed in such a manner that arrangement of the respective sound emitting modules corresponds to the positions of left and right ears of the user. On the remaining second frames F2, which are installed in positions lower than that of the above second frame F2, plural vibrating and light-emitting modules M2 are installed. Further, a second frame F2 extends toward positions where arms of a user are put when the user is supported by the human body support device 1, and the second frame F2 is also provided with plural vibrating and light-emitting modules M2.

All of the respective modules M1 and M2 have disk shapes and have the same color. As a result, a sense of unity in design is prepared and presented; however, it is not required to have the above construction, and the external appearance of each module may be different from those of others. Further, the size of each sound emitting module M1 is approximately the same as those of other sound emitting modules M1, and the size of each vibrating and light-emitting module M2 is also approximately the same as those of other vibrating and light-emitting modules M2; and the sound emitting module M1 is slightly larger than the vibrating and light-emitting module M2. As a result, an accent in design is prepared and presented; however, it is not required to have the above construction, and the shapes of the respective modules M1 and M2 can be determined arbitrarily.

Further, the above modules M1 and M2 may comprise elastic parts which are arranged in positions where the body of a user is brought in contact with the modules, and are constructed by using elastic material such as urethane, sponge, fibers, or the like. The direction and the position of each of the modules M1 and M2 are designed in such a manner that the module is brought in contact with the body of a user in an appropriate manner, so as to support the body of the user in a comfortable and pleasant manner. The thickness and the strength of elastic force of each module may be set to be the same as those of other modules, or may be set to be different from those of other modules according to respective parts of a user's body which are to be brought in contact with the respective modules. Further, the respective modules M1 and M2 are arranged in the frame F by setting the position and the direction of each module in such a manner that the respective modules do not come in contact with each other. Thus, effect of resonance between modules due to sound emission and/or vibration is eliminated.

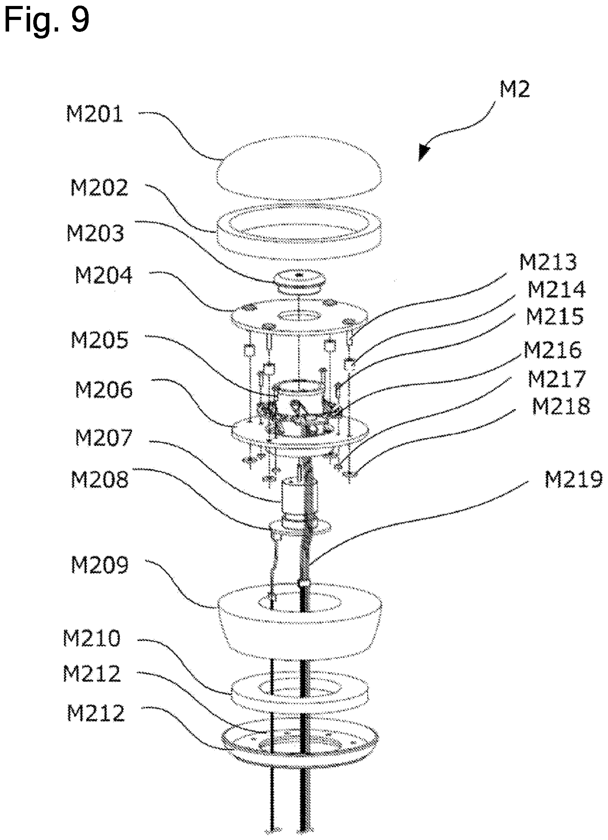

Next, the construction of the vibrating and light-emitting module M2 will be explained with reference to Fig. 9. The vibrating and light-emitting module M2 comprises a construction in which an upper urethane cushion M201 and a ring urethane cushion M202, an upper cover M203, an inner upper cover M204, a vibrator cover M205, an LED circuit board M216, an inner lower cover M206, a voice coil motor M207 functioning as a vibrator, a vibrator control board M208, an inner urethane cushion M209, a wood ring M210, and a bottom face cover M212 are stacked. The inner upper cover M204, the vibrator cover M205, the LED circuit board M216, and the inner lower cover M206 are fixed by inner-cover fixing screws M213, spacers M214, LED-circuit-board fixing screws M215, LED-circuit-board fixing nuts M217, and inner-cover fixing nuts M218. Power-supplying/controlling wires M219 for an LED are connected to the LED circuit board M216, and power-supplying/signaling wires M211 for a vibrator are connected to the vibrator control board M208. Further, the upper urethane cushion M201, the ring urethane cushion M202, the upper cover M203, the inner upper cover M204, the vibrator cover M205, the LED circuit board M216, the inner lower cover M206, the voice coil motor M207, the vibrator control board M208, the inner urethane cushion M209, and the wood ring M210, as a whole, are wrapped by a mesh sheet (cloth and so on) which is not shown in the figure. The end part of the sheet is inserted between the wood ring M210 and the bottom face cover M212 and fixed thereby.

The upper urethane cushion M201 and the ring urethane cushion M202 function as elastic parts which are used when supporting the body of a user. On the other hand, the inner urethane cushion M209, which is positioned to surround the voice coil motor M207 and has an annular shape, bears responsibility for assistance for keeping elasticity when supporting the body of a user, and attenuating of vibration applied from the voice coil motor M207 to the frame F to suppress propagation of the vibration, to prevent occurrence of resonance due to propagation of vibration from the voice coil motor M207 to other modules via the frame F. That is, the above construction, wherein the voice coil motor M207 is covered by the annular-shape inner urethane cushion M209, functions as a vibration suppressing mechanism which suppresses propagation of vibration from the voice coil motor M207 to the frame F.

It is desirable that the respective modules M1 and M2 are constructed to be easily attached/detached to/from the frame F by a user. As a result, it becomes possible to detach modules M1 and M2 from the flame F by a user for replacement thereof, or additionally attach modules M1 and M2 to free spaces in the frame F. Especially, it is desirable that the vibrating and light-emitting modules M2 installed in positions, where soles of feet of a user are brought in contact with the modules, are constructed to be attachable/detachable. As a result, it becomes possible to attach/detach a vibrating and light-emitting module M2 which is to be brought in contact with a sole of a foot, and/or replace a vibrating and light-emitting modules M2 to a different vibrating and light-emitting modules M2 which has a size in a thickness direction different from that of the former, to make the module fit the height of a user.

As explained above, the CPU 101 in the information processing device 10 may control respective LEDs 30 in the LED group or respective voice coil motors 50 in the voice coil motors group in different manners. For example, the CPU 101 in the information processing device 10 can perform controlling of vibration with respect to one voice coil motor 50 and controlling of vibration with respect to the other voice coil motor 50, separately. Thus, in the above-explained first operation mode, for example, it is possible to make the vibration frequency and/or the amplitude with respect to a vibrating and light-emitting module M2 in a position corresponding to the position of the waist of a user different from the vibration frequency and/or the amplitude with respect to a vibrating and light-emitting module M2 in a position corresponding to the position of an arm of the user. As a result, it becomes possible to provide a user with a new sensation that has not yet experienced, compared with the case that the modes of vibration of the respective vibrating and light-emitting modules M2 are set to be the same with each other. Similarly, regarding light emission, the CPU 101 in the information processing device 10 can perform controlling of light emission with respect to one LED 30 and controlling of light emission with respect to the other LED 30, separately. Thus, in the above-explained first operation mode, for example, it is possible to make the vibration frequency and/or the amplitude with respect to a vibrating and light-emitting module M2 in one position different from the vibration frequency and/or the amplitude with respect to a vibrating and light-emitting module M2 in the other position. As a result, it becomes possible to provide a user with a new sensation that has not yet experienced, compared with the case that the modes of light emission of the respective vibrating and light-emitting modules M2 are set to be the same with each other.

According to the embodiment explained above, it is possible to provide a user with a new sensation, through a tactile sensation, an auditory sensation, and a visual sensation.

### [Modification Examples]

The present invention is not limited by the above explained embodiment. The above-explained embodiment may be modified as explained below. Further, two or more modification examples explained below may be combined and implemented.

The vibrating and light-emitting module M2 may be operated to vibrate or emit light, in the state that it is being detached from the frame F. In the above case, the vibrating and light-emitting module M2, which is detachable from the frame F, comprises a battery as an electric power supplying source. Further, the vibrating and light-emitting module M2 comprises a control device for autonomously controlling vibration and light emission in place of the information processing device 10, or a control device for receiving control data from the information processing device 10, via data communication in wireless manner or the like, for controlling vibration and light emission. That is, in the present invention, at least one or more vibrating and light-emitting modules M2 are attachable/detachable to/from the frame F; and, regarding the above vibrating and light-emitting module M2, when the above module is in the state that it is being connected to the frame F, the voice coil motor 50 vibrates or the LED 30 emits light by using electric power supplied via the frame F, and, when the above module is in the state that it is being detached from the frame F, the voice coil motor 50 vibrates or the LED 30 emits light by using electric power supplied from a battery included in the above module. As a result, the vibrating and light-emitting module M2 is made to be able to perform vibration and light emission operation as a stand alone module after it is detached from the frame F; so that, for example, a user whose body is being supported by the human body support device 1 and a user whose body is not being supported by the human body support device 1 can have experience of sharing, between the users, a stimulation through a tactile sensation or visual sensation.

In the embodiment, the number of the sound emitting modules M1 is two, i.e., one each for the left side and the right side; however, the construction is not limited to the above construction, so that plural speakers for the left side and the right side may be included in a single sound emitting module M1, or a speaker may be installed in each of three or more sound emitting modules M1 (typically, the number of the modules is an even number). That is, in the present invention, each of at least one or more sound emitting modules is provided with a speaker, and each of at least two or more vibrating and light-emitting modules is provided with a voice coil motors and an LED.

The size, shape, direction, position, or arrangement of each of the above-explained modules M1 and M2 is a mere example thereof, and tangible embodiments of the respective modules are not limited to the examples explained above.

The human body support device 1 according to the above embodiment has a shape that is appropriate for use as a chair or a couch; however, as long as the body of a user can be supported, the shape is not limited to the above shape. For example, the shape may be a shape similar to that of a bed. Further, the human body support device 1 may not be limited to that for use in a home, and may be that loaded on a moving body such as an automobile, a train, or an aircraft or the like, and used therein.

The human body support device 1 may further comprise a video reproducing device such as a head mounted display or the like. In such a case, video reproduction by the video reproducing device, sound emission by the group of speakers 70, and vibration by the group of voice coil motors 50 are synchronously controlled by the CPU 101.

Plural human body support devices 1 may be used to share stimulus, that is applied to a body, by plural users. Specifically, one human body support device 1 positioned in a first location and the other human body support device 1 positioned in a second location are communicably connected with each other via a wired or wireless network, and an information processing device 10 may perform, via the network, control that is in common in the respective human body support devices 1 and relates to sound emission, vibration, and light emission.

The present invention may be provided as an information processing method comprising steps of the process performed in the information processing device 10. Further, the present invention may be provided as a program executed in the information processing device 10. The above program may be provided as that having a form of a storage medium such as an optical disk or the like which stores the program, a form wherein the program is downloaded to a computer via a network such as the Internet or the like and installed in the computer to make the program usable, or the like.

In the above description, the present invention has been explained in detail, and, in this regard, it is obvious to a person skilled in the art that the present invention is not limited to the embodiment explained in the present specification. The present invention can be implemented as a rectified or modified embodiment without departing from the gist and scope of the present invention defined by the descriptions in the claims. That is, the description in the present specification is provided for the purpose of explanation of examples, and is not that intended to be used to limit the present invention.

### REFERENCE SIGNS LIST

1 ... Human body support device: 10 ... Information processing device: 20 ... Control device: 30 ... LED (group): 40 ... Amplifier: 50, M207 ... Voice coil motor (group): 60 ... Amplifier: 70 ... Speaker (group): 101 ... CPU: 102 ... RAM: 103 ... ROM: 104 ... Auxiliary storage device: 105 ... Communication IF: 106 ... Display unit: 107 ... Manipulation unit: M1 ... Sound emitting module: M2 ... Vibrating and light-emitting module: F ... Frame: F1 ... First frame: F2 ... Second frame: H ... User: M201 ... Upper urethane cushion: M202 ... Ring urethane cushion: M203 ... Upper cover: M204 ... Inner upper cover: M205 ... Vibrator cover: M206 ... Inner lower cover: M207 ... Voice coil motor: M208 ... Vibrator control board: M209 ... Inner urethane cushion: M210 ... Wood ring: M211 ... Power-supplying/signaling wires for a vibrator: M212 ... Bottom face cover: M213 ... Screw for fixing inner cover: M214 ... Spacer: M216 ... LED circuit board: M216 ... Screw for fixing LED circuit board: M217 ... Nut for fixing LED circuit board: M218 ... Nut for fixing inner cover: M219... Power-supplying/controlling wires for an LED

## Claims

1. A human body support device **characterized in that** it comprises:
a frame; and
plural modules which are arranged in plural parts, in the frame, which support a body of a user;
wherein
each of the plural modules comprises an elastic part which is arranged in a position where the body of the user is brought in contact with the module; and
in the plural modules,
each of at least one or more sound emitting modules is provided with a sound emitting device, and
each of at least two or more vibrating and light-emitting modules is provided with a vibrating device and a light emitting device.

2. The human body support device as recited in Claim 1 **characterized in that**:
the human body support device comprises an information processing device which controls the sound emitting device, the vibrating device, and the light emitting device in a synchronizing manner.

3. The human body support device as recited in Claim 2 **characterized in that**:
the information processing device controls the vibrating device or the light emitting device to vibrate or emit light in different manners.

4. The human body support device as recited in Claim 2 or 3 **characterized in that**:
the information processing device performs switching of a mode between a first operation mode for performing light emission accompanied with vibration in a state that the body of a user is being supported, and a second operation mode for performing light emission without vibration in a state that the body of a user is not being supported.

5. The human body support device as recited in any one of Claims 1 to 4 **characterized in that**:
each of the vibrating and light-emitting modules comprises a vibration suppressing mechanism for suppressing propagation of vibration from the vibrating device installed in the vibrating and light-emitting module to the frame.

6. The human body support device as recited in any one of Claims 1 to 5 **characterized in that**:
the respective modules are arranged in such a manner that, in the frame, the respective modules do not come in contact with each other.

7. The human body support device as recited in any one of Claims 1 to 6 **characterized in that**:
the frame comprises a first frame which extends in the direction that the backbone of a user extends when the body of the user is supported, and plural second frames which intersect the first frame, and
the respective modules are arranged in the second frames.

8. The human body support device as recited in any one of Claims 1 to 7 **characterized in that**:
the at least one or more vibrating and light-emitting modules are attachable/detachable to/from the frame; and
in the vibrating and light-emitting module,
when the vibrating and light-emitting module is in the state that it is being connected to the frame, the vibrating device performs vibration or the light emitting device performs light emission by using electric power supplied via the frame, and
when the vibrating and light-emitting module is in the state that it is being detached from the frame, the vibrating device performs vibration or the light emitting device performs light emission by using electric power supplied from a battery included in the vibrating and light-emitting module.

9. The human body support device as recited in any one of Claims 1 to 8 **characterized in that**:
the shapes of the respective modules are approximately the same with each other.

10. The human body support device as recited in any one of Claims 1 to 9 **characterized in that**:
the sizes of the respective vibrating and light-emitting modules are approximately the same with each other.
